(19) Europäisches Patentamt
European Patent Office
Office européen des brevets

(11) Veröffentlichungsnummer: **0 169 815**
**B1**

(12) # EUROPÄISCHE PATENTSCHRIFT

(45) Veröffentlichungstag der Patentschrift: **02.11.88**

(51) Int. Cl.⁴: **C 07 D 239/46**, C 07 D 239/47, C 07 D 239/48, C 07 D 251/16, C 07 D 251/26, A 01 N 47/36

(21) Anmeldenummer: **85810336.9**

(22) Anmeldetag: **22.07.85**

(54) **N-Arylsulfonyl-N'-triazinyl- und -pyrimidinylharnstoffe.**

(30) Priorität: **26.07.84 CH 3621/84**

(43) Veröffentlichungstag der Anmeldung: **29.01.86 Patentblatt 86/5**

(45) Bekanntmachung des Hinweises auf die Patenterteilung: **02.11.88 Patentblatt 88/44**

(84) Benannte Vertragsstaaten: **AT BE CH DE FR GB IT LI NL**

(56) Entgegenhaltungen:
**EP-A-0 094 790**
**EP-A-0 098 569**
**EP-A-0 102 925**
**EP-A-0 108 708**
**DE-A-3 234 448**
**GB-A-2 126 586**
**US-A-4 452 628**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**

**EP 0 169 815 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue, herbizid wirksame und pflanzenwuchsregulierende N-Arylsulfonyl-N'-triazinyl- und -pyrimidinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue Aminopyrimidine und Aminotriazine.

Die erfindungsgemässen N-Arylsulfonyl-N'-triazinyl- und -pyrimidinylharnstoffe entsprechen der allgemeinen Formel I

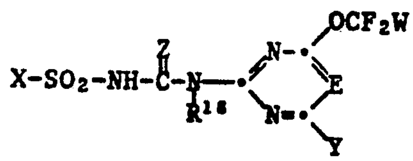 (I),

worin

X einen Rest der Formel 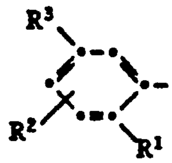 oder 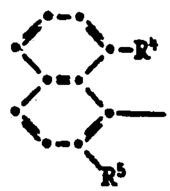 ,

Y $C_1$-$C_3$-Alkyl, $C_1$-$C_3$-Halogenalkyl, $C_1$-$C_3$-Alkoxy, $C_1$-$C_3$-Halogenalkoxy, $C_2$-$C_3$-Alkoxyalkyl, $C_1$-$C_3$-Alkylthio, Halogen oder $-NR^{16}R^{17}$,
Z Sauerstoff oder Schwefel,
E Stickstoff oder die Methinbrücke -CH=,
W Chlor oder Brom,
$R^1$ Wasserstoff, Halogen, Cyan, Nitro, $C_1$-$C_4$-Halogenalkyl, $C_1$-$C_4$-Alkyl, $C_1$-$C_4$-Alkoxy, $-CO-R^6$, $-NR^7R^8$, $-S(O)_m$-$C_1$-$C_4$-Alkyl oder $-SO_2R^9$,
$R^2$ Wasserstoff, Fluor, Chlor, Brom, Nitro, Trifluormethyl, $-NR^{20}R^{21}$, Methyl, Äthyl, Methoxy, Äthoxy oder $-S(O)_m$-$C_1$-$C_4$-Alkyl,
$R^3$ Wasserstoff, Fluor, Chlor, Brom, Amino, Nitro oder Methoxy,
$R^6$ Wasserstoff, $C_1$-$C_4$-Alkyl, $C_3$-$C_3$-Alkenyloxy, $C_3$-$C_5$-Alkinyloxy, $C_1$-$C_4$-Halogenalkyl, $C_1$-$C_5$-Alkylthio, Phenoxy, Benzyloxy, $-NR^{10}R^{11}$ oder gegebenenfalls durch 1 - 3 Halogenatome oder $C_1$-$C_3$-Alkoxy substituiertes $C_1$-$C_5$-Alkoxy,
$R^7$ Wasserstoff, Methoxy, Äthoxy, $C_1$-$C_4$-Alkyl oder $-CO-R^{12}$,
$R^8$ Wasserstoff oder $C_1$-$C_4$-Alkyl,
$R^9$ eine Gruppe $-O-R^{13}$ oder $-NR^{14}R^{15}$,
$R^{13}$ gegebenenfalls durch 1 - 3 Halogenatome substituiertes $C_1$-$C_4$-Alkyl, Phenyl oder Benzyl,
$R^{18}$ Wasserstoff, $C_1$-$C_3$-Alkyl oder $C_1$-$C_3$-Alkoxy und
m die Zahlen Null, eins oder zwei bedeuten,

wobei

$R^4$ die gleiche Bedeutung wie $R^2$;
$R^5$ die gleiche Bedeutung wie $R^1$;
$R^{10}$, $R^{11}$, $R^{14}$ und $R^{20}$ die gleiche Bedeutung wie $R^7$; und
$R^{12}$, $R^{15}$, $R^{16}$, $R^{17}$ und $R^{21}$ die gleiche Bedeutung wie $R^8$ haben;
sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 9419, 30 141 oder 84 020 beschrieben.

In den Definitionen ist unter Alkyl geradkettiges oder verzweigtes Alkyl zu verstehen; z. B. Methyl, Äthyl, n-Propyl, i-Propyl oder die vier isomeren Butyl.

Unter Alkoxy ist zu verstehen: Methoxy, Äthoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Äthoxy oder i-Propyloxy.

Beispiele für Alkylthio sind Methylthio, Äthylthio, n-Propylthio, i-Propylthio, n-Butylthio oder n-Pentylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl oder 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl oder n-Propylsulfonyl, insbesondere aber Methylsulfonyl und Äthylsulfonyl.

Unter Halogen in den Definitionen sowie als Teil in Halogenalkoxy sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl sowie isomere Pentinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel I sind die Verbindungen bevorzugt, in denen entweder

a) X den gegebenenfalls substituierten Phenylrest oder
b) Y einen Rest mit höchstens 2 Kohlenstoffatomen oder
c) Z Sauerstoff oder
d) E die Methinbkrücke =CH- oder
e) W Brom oder
f) $R^3$ und $R^4$ Wasserstoff oder
g) $R^{18}$ Wasserstoff oder Methyl bedeuten.

Durch Kombination einiger bevorzugter Merkmale ergibt sich folgende weiter bevorzugte Gruppe von Verbindungen der Formel I, in denen

X den gegebenenfalls substituierten Phenylrest,
Y einen Rest mit höchstens 2 Kohlenstoffatomen,
Z Sauerstoff und
$R^3$ und $R^{18}$ Wasserstoff

bedeuten.

Innerhalb dieser Gruppe sind weiterhin die Verbindungen bevorzugt, in denen $R^2$ für Wasserstoff steht.

Eine besonders hervorzuhebende Untergruppe der Verbindungen der Formel I zeichnet sich dadurch aus, dass

X den gegebenenfalls substituierten Phenylkern,
Y einen Rest mit höchstens 2 Kohlenstoffatomen,
Z Sauerstoff,
E die Methingruppe,
W Brom und
$R^{18}$ Wasserstoff bedeuten,
wobei

$R^1$ für Wasserstoff, Halogen, Nitro, $C_1$-$C_4$-Halogenalkyl, $C_1$-$C_4$-Alkoxy, $C_1$-$C_4$-Alkyl, $-S(O)_m$-$C_1$-$C_4$-Alkyl, $-SO_2-N(CH_3)_2$, $-SO_2OCH_2CF_3$ oder -CO-$R^6$,

$R^2$ für Wasserstoff, Fluor, Chlor, Nitro, Amino, Trifluormethyl, Methyl, Methoxy, Äthoxy oder $-S(O)_m$-$C_1$-$C_4$-Alkyl,

$R^3$ für Wasserstoff, Fluor, Chlor, Brom, Amino, Nitro oder Methoxy,

$R^6$ für Wasserstoff, Methyl, $C_3$-$C_5$-Alkenyloxy, $C_3$-$C_5$-Alkinyloxy, $C_1$-$C_3$-Alkylthio, Dimethylamino, Methylamino, Amino oder gegebenenfalls durch 1 bis 3 Halogenatome oder $C_1$-$C_3$-Alkoxy substituiertes $C_1$-$C_5$-

Alkoxy und

m für die Zahlen Null, eins oder zwei stehen.

Als bevorzugte Einzelverbindungen sind zu nennen:

N-(2-Methoxycarbonylphenyl-sulfonyl)-N'-(4-difluorbrommethoxy-6-methoxypyrimidin-2-yl)-harnstoff,
N-(2-Methoxycarbonylphenyl-sulfonyl)-N'-(difluorbrommethoxy-4-methylpyrimidin-2-yl)-harnstoff und
N-(2-Nitrophenyl-sulfonyl)-N'-(4-diflurobrommethoxy-6-methylpyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Arylsulfonamid der Formel II

$$X - SO_2 - NH_2 \qquad (II),$$

worin X die unter Formel I gegebene Bedeutung hat, in Gegenwart einer Base mit einem N-Triazinyl- oder N-Pyrimidinylcarbamat der Formel III

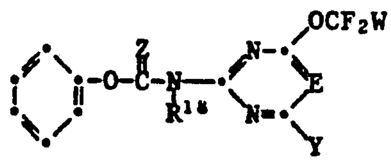
(III),

worin $R^{18}$, E, W, Y und Z die unter Formel I gegebene Bedeutung haben, umsetzt.

Nach einem zweiten Verfahren gelangt man zu Verbindungen der Formel I, indem man ein Arylsulfonylisocyanat oder -isothiocyanat der Formel IV

$$X - SO_2 - N = C = Z \qquad (IV),$$

worin X und Z die unter Formel I gegebene Bedeutung haben, gegebenenfalls in Gegenwart einer Base, mit einem Aminopyrimidin oder -triazin der Formel V

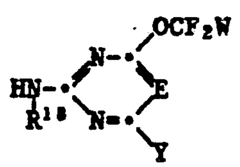
(V),

worin $R^{18}$, E, W und Y die unter Formel I gegebene Bedeutung haben, umsetzt.

Nach einem weiteren Verfahren werden die Verbindungen der Formel I, in denen $R^{18}$ Wasserstoff bedeutet, hergestellt, indem man ein Arylsulfonamid der oben angegebenen Formel II gegebenenfalls in Gegenwart einer Base mit einem Isocyanat oder Isothiocyanat der Formel VI

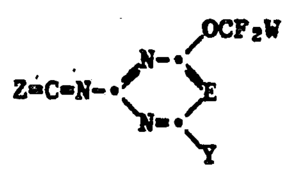
(VI),

worin E, W, Y und Z die unter Formel I gegebene Bedeutung haben, umsetzt.

Schliesslich kann man die Verbindungen der Formel I auch erhalten, indem man ein N-Arylsulfonylcarbamat der Formel VII

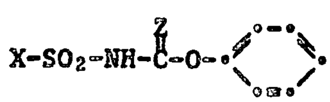
(VII)

worin X und Z die unter Formel I gegebene Bedeutung haben, mit einem Aminopyrimidin- oder -triazin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können mit Vorteil bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Als Basen können sowohl organische Basen wie Amine, wie Triäthylamin, Chinuclidin, Pyridin etc. als auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte können durch Einengen und/oder Verdampfen des Lösungsmittels isoliert und durch Umkristallisieren oder Zerreiben des festen Rückstandes in Lösungsmitteln, in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Die Zwischenprodukte der Formeln II, IV und VII sind bekannt oder können analog zu bekannten Verfahren hergestellt werden.

Die Zwischenprodukte der Formel V sind neu. Sie wurden speziell für die Synthese der Wirkstoffe der Formel I entwickelt und bilden daher einen weiteren Gegenstand der vorliegenden Erfindung.

Die Verbindungen der Formel V werden erhalten, indem man ein Aminopyrimidin oder -triazin der Formel VIII

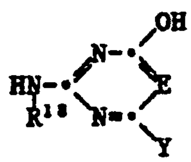

(VIII),

worin E, $R^{18}$ und Y die unter Formel I gegebene Bedeutung haben, in Gegenwart einer Base mit Difluorbromchlormethan oder Difluordibrommethan umsetzt.

Das Verfahren zur Herstellung der Verbindungen der Formel V wird mit Vorteil in einem inerten polaren Lösungsmittel oder Lösungsmittelgemisch ausgeführt. Geeignete Lösungsmittel sind Äther wie Dioxan, Tetrahydrofuran, Äthylenglykoldimethyläther, Diäthylenglykoldimethyläther; Alkohole wie Methanol, Äthanol; Ketone wie Aceton, Äthylmethylketon; Dimethylformamid; Acetonitril oder Dimethylsulfoxid. Als Basen sind in besonderer Weise geeignet: Natrium- und Calciumhydrid, Kalium- und Natriumhydroxid, Kalium- und Natriumcarbonat. In geeigneten Fällen kann die Base als wässrige Lösung zugesetzt werden.

Die Ausgangsmaterialien der Formel VIII sind bekannt oder werden analog zu bekannten Verfahren erhalten.

Die Zwischenprodukte der Formeln III und VI sind ebenfalls neu. Sie können analog zu bekannten Verfahren aus den Zwischenprodukten der Formel V hergestellt werden.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Zuckerrohr, Baumwolle, Soja, Mais und Reis, vorzugsweise aber Getreide befähigen. Es werden dabei teilweise auch Unkräuter geschädigt, welchen bisher nur mit Totalherbiziden beizukommen war.

Die Wirkungsart dieser Stoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das negetative Wachstum eingeschränkt wird.

Bei monokotylen Pflanzen, z. B. Gräsern oder auch Kulturpflanzen wie Getreide, ist eine Hemmung des vegetativen Wachstums manchmal gewünscht und vorteilhaft. Eine derartige Wuchshemmung ist unter

anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis

10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol Äthylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

| | |
|---|---|
| <u>Emulgierbare Konzentrate</u>: | |
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |
| <u>Stäube</u>: | |
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |
| <u>Suspensions-Konzentrate</u>: | |
| Aktiver Wirkstoff: | 5 bis 75 %, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |
| <u>Benetzbares Pulver</u>: | |
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 5 bis 90 % |
| <u>Granulate</u>: | |
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Herstellungsbeispiele**

**Beispiel H 1**: <u>N-(2-Methoxycarbonylphenyl-sulfoyl)-N'-(4-difluorbrommethoxy-6-methoxypyrimidin-2-yl)-harnstoff</u>

a) 7,1 g 2-Amino-4-hydroxy-6-methoxypyrimidin, 7,3 g Kaliumcarbonat und 20,8 g Dibromdifluormethan werden in 50 ml Dimethylformamid im Bombenrohr für 10 Std. bei einer Temperatur von 80°C geschüttelt. Das

Dimethylformamid wird abgedampft und der Rückstand in Essigester/Wasser aufgenommen. Die organische Phase wird abgetrennt, eingedampft und der Rückstand an Kieselgel mit Toluol/Essigester (9 : 1) chromatographisch gereinigt. So erhält man 2-Amino-4-difluorbrommethoxy-6-methoxypyrimidin als farbloses Öl, welches direkt weiter verarbeitet wird.

b) 1,3 g 2-Amino-4-difluorbrommethoxy-6-methoxypyrimidin und 1,16 g 2-Methoxycarbonylphenylsulfonylisocyanat werden in 20 ml Methylenchlorid für 15 Std. bei Raumtemperatur gerührt und dann eingeengt. Durch Chromatographieren des Rückstandes an Kieselgel mit Essigester/Hexan (1 : 1) erhält man 1,4 g N-(2-Methoxycarbonyl-phenylsulfonyl)-N'-(4-difluorbrommethoxy-6-methoxypyrimidin-2-yl)-harnstoff mit einem Schmelzpunkt von 144-145° C.

In analoger Weise erhält man die in den anschliessenden Tabellen aufgeführten Zwischen- und Endprodukte.

**Tabelle 1:**

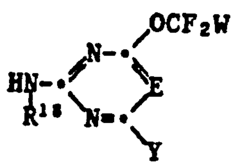

| Verb.Nr. | $R^{18}$ | W | Y | E | Smp. [° C] |
|---|---|---|---|---|---|
| 1.01 | H | Br | $CH_3$ | CH | 112-113 |
| 1.02 | H | Cl | $CH_3$ | CH | |
| 1.03 | H | Br | $OCH_3$ | CH | Öl |
| 1.04 | H | Cl | $OCH_3$ | CH | |
| 1.05 | H | Br | $OC_2H_5$ | CH | |
| 1.06 | H | Cl | $OC_2H_3$ | CH | |
| 1.07 | H | Br | $CH_2OCH_3$ | CH | |
| 1.08 | H | Cl | $CH_2OCH_3$ | CH | |
| 1.09 | H | Br | Cl | CH | |
| 1.10 | H | Cl | Cl | CH | |
| 1.11 | H | Br | $N(CH_3)_2$ | CH | |
| 1.12 | H | Cl | $N(CH_3)_2$ | CH | |
| 1.13 | $CH_3$ | Br | $OCH_3$ | CH | |
| 1.14 | $CH_3$ | Cl | $OCH_3$ | CH | |
| 1.15 | H | Br | $OCH_3$ | N | |
| 1.16 | H | Cl | $OCH_3$ | N | |
| 1.17 | H | Br | $CH_3$ | N | |
| 1.18 | H | Cl | $CH_3$ | N | |

**Tabelle 2:**

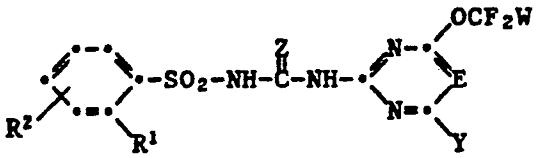 (III),

| Verb.Nr | $R^1$ | $R^2$ | Y | Z | W | E | Smp. [°C] |
|---|---|---|---|---|---|---|---|
| 2.01 | $COOCH_3$ | H | $CH_3$ | O | Br | CH | 164-165 |
| 2.02 | $COOCH_3$ | H | $OCH_3$ | O | Br | CH | 144-145 |
| 2.03 | $COOCH_3$ | H | $OCH_3$ | O | Cl | CH | |
| 2.04 | $COOCH_3$ | H | $CH_3$ | O | Cl | CH | |
| 2.05 | $COOCH_3$ | H | $C_2H_5$ | O | Br | CH | |
| 2.06 | $COOCH_3$ | H | $C_2H_5$ | O | Cl | CH | |
| 2.07 | $COOCH_3$ | H | $CH_2F$ | O | Br | CH | |
| 2.08 | $COOCH_3$ | H | $CH_2F$ | O | Cl | CH | |
| 2.09 | $COOCH_3$ | H | $N(CH_3)_2$ | O | Cl | CH | |
| 2.10 | $COOCH_3$ | H | $N(CH_3)_2$ | O | Br | CH | |
| 2.11 | $COOCH_3$ | H | $OC_2H_5$ | O | Br | CH | |
| 2.12 | $COOCH_3$ | H | $OC_2H_5$ | O | Cl | CH | |
| 2.13 | $COOCH_3$ | H | $OCH_3$ | O | Br | N | |
| 2.14 | $COOCH_3$ | H | $CH_3$ | O | Br | N | |
| 2.15 | $COOCH_3$ | H | $CH_3$ | O | Cl | N | |
| 2.16 | $COOCH_3$ | H | $OCH_3$ | O | Cl | N | |
| 2.17 | $COOC_2H_5$ | H | $OCH_3$ | O | Br | CH | |
| 2.18 | $COOC_2H_5$ | H | $CH_3$ | O | Br | CH | |
| 2.19 | $COOC_2H_5$ | H | $CH_3$ | O | Cl | CH | |
| 2.20 | $COOC_2H_5$ | H | $OCH_3$ | O | Cl | CH | |
| 2.21 | Cl | H | $OCH_3$ | O | Cl | CH | |
| 2.22 | Cl | H | $CH_3$ | O | Cl | CH | |
| 2.23 | Cl | H | $CH_3$ | O | Br | CH | |
| 2.24 | Cl | H | $OCH_3$ | O | Br | CH | |
| 2.25 | $OCH_3$ | H | $CH_3$ | O | Br | CH | |
| 2.26 | $OCH_3$ | H | $OCH_3$ | O | Br | CH | |
| 2.27 | $OCH_3$ | H | $OCH_3$ | O | Cl | CH | |
| 2.28 | $OCH_3$ | H | $CH_3$ | O | Cl | CH | |
| 2.29 | $OC_2H_5$ | H | $CH_3$ | O | Cl | CH | |
| 2.30 | $OC_2H_5$ | H | $OCH_3$ | O | Cl | CH | |
| 2.31 | $OC_2H_5$ | H | $OCH_3$ | O | Br | CH | |
| 2.32 | $OC_2H_5$ | H | $CH_3$ | O | Br | CH | |
| 2.33 | $NO_2$ | H | $CH_3$ | O | Br | CH | 159-160 |
| 2.34 | $NO_2$ | H | $OCH_3$ | O | Br | CH | |
| 2.35 | $NO_2$ | H | $OCH_3$ | O | Cl | CH | |
| 2.36 | $NO_2$ | H | $CH_3$ | O | Cl | CH | |
| 2.37 | $SO_2N(CH_3)_2$ | H | $CH_3$ | O | Cl | CH | |
| 2.38 | $SO_2N(CH_3)_2$ | H | $OCH_3$ | O | Cl | CH | |
| 2.39 | $SO_2N(CH_3)_2$ | H | $OCH_3$ | O | Br | CH | |

| | | | | | | |
|---|---|---|---|---|---|---|
| 2.40 | $SO_2N(CH_3)_2$ | H | $CH_3$ | O | Br | CH |
| 2.41 | $SO_2CH_3$ | H | $CH_3$ | O | Br | CH |
| 2.42 | $SO_2CH_3$ | H | $OCH_3$ | O | Br | CH |
| 2.43 | $SO_2CH_3$ | H | $OCH_3$ | O | Cl | CH |
| 2.44 | $SO_2CH_3$ | H | $CH_3$ | O | Cl | CH |
| 2.45 | $SCH_3$ | H | $CH_3$ | O | Cl | CH |
| 2.46 | $SCH_3$ | H | $OCH_3$ | O | Cl | CH |
| 2.47 | $SCH_3$ | H | $OCH_3$ | O | Br | CH |
| 2.48 | $SCH_3$ | H | $CH_3$ | O | Br | CH |
| 2.49 | Br | H | $CH_3$ | O | Br | CH |
| 2.50 | Br | H | $OCH_3$ | O | Br | CH |
| 2.51 | Br | H | $OCH_3$ | O | Cl | CH |
| 2.52 | Br | H | $CH_3$ | O | Cl | CH |
| 2.53 | $-(CH_2)_2-CF_3$ | H | $CH_3$ | O | Cl | CH |
| 2.54 | $-(CH_2)_2-CF_3$ | H | $OCH_3$ | O | Cl | CH |
| 2.55 | $-(CH_2)_2-CF_3$ | H | $OCH_3$ | O | Br | CH |
| 2.56 | $-(CH_2)_2-CF_3$ | H | $CH_3$ | O | Br | CH |
| 2.57 | $CF_3$ | H | $OCH_3$ | O | Cl | CH |
| 2.58 | $CF_3$ | H | $CH_3$ | O | Cl | CH |
| 2.59 | $CF_3$ | H | $CH_3$ | O | Br | CH |
| 2.60 | $CF_3$ | H | $OCH_3$ | O | Br | CH |
| 2.61 | F | H | $OCH_3$ | O | Br | CH |
| 2.62 | F | H | $CH_3$ | O | Br | CH |
| 2.63 | F | H | $CH_3$ | O | Cl | CH |
| 2.64 | F | H | $OCH_3$ | O | Cl | CH |
| 2.65 | $CH_3$ | H | $OCH_3$ | O | Cl | CH |
| 2.66 | $CH_3$ | H | $CH_3$ | O | Cl | CH |
| 2.67 | $CH_3$ | H | $CH_3$ | O | Br | CH |
| 2.68 | $CH_3$ | H | $OCH_3$ | O | Br | CH |
| 2.69 | $CH_2Cl$ | H | $OCH_3$ | O | Br | CH |
| 2.70 | $CH_2Cl$ | H | $CH_3$ | O | Br | CH |
| 2.71 | $CH_2Cl$ | H | $CH_3$ | O | Cl | CH |
| 2.72 | $CH_2Cl$ | H | $OCH_3$ | O | Cl | CH |
| 2.73 | $SO_2-C_3H_7-n$ | H | $OCH_3$ | O | Cl | CH |
| 2.74 | $SO_2-C_3H_7-n$ | H | $CH_3$ | O | Cl | CH |
| 2.75 | $SO_2-C_3H_7-n$ | H | $CH_3$ | O | Br | CH |
| 2.76 | $SO_2-C_3H_7-n$ | H | $OCH_3$ | O | Br | CH |
| 2.77 | $OC_3H_7-n$ | H | $OCH_3$ | o | Br | CH |
| 2.78 | $OC_3H_7-n$ | H | $CH_3$ | O | Br | CH |
| 2.79 | $OC_3H_7-n$ | H | $CH_3$ | O | Cl | CH |
| 2.80 | $OC_3H_7-n$ | H | $CH_3$ | O | Cl | CH |

**Formulierungsbeispiele:**

**Beispiel F 1**: Formulierungsbeispiele für Wirkstoffe der Formel I (% = Gewichtsprozent)

| a) Spritzpulver | a) | b) | c) |
|---|---|---|---|
| Wirkstoff | 20 % | 50 % | 0,5 % |
| Na-Ligninsulfonat | 5 % | % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Äthylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %-ige wässrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

**Biologische Beispiele:**

**Beispiel B 1**: Herbizidwirkung vor dem Auflaufen der Pflanzen

Kunststofftöpfe werden mit expandiertem Vermiculit (Dichte: 0,135 g/cm$^3$, Wasserabsorptionsvermögen:

0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Digitaria sanguinalis und Stellaria media. Die Versuchsgefässe werden anschliessend in einer Klimakammer bei 20°C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5 Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze Wirkstoff Nr | Nasturtium | Stellaria | Agrostis | Digitaria |
|---|---|---|---|---|
| 2.01 | 2 | 1 | 2 | 2 |
| 2.02 | 1 | 1 | 1 | 1 |
| 2.33 | 1 | 1 | 1 | 1 |

**Beispiel B 2**: Nachweis der Wuchshemmung bei tropischen Bodenbedeckern-Leguminosen (cover crops)

Die Versuchspflanzen (Phosphocarpus palustris und Centrosema pubescens) werden bis zum ausgewachsenen Stadium herangezogen und bis auf eine Höhe von 15 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen).

**Beispiel B 3**: Wuschregulierung an Sojabohnen

In Kunststoffbehältern mit einem Erde-Torf-Sandgemisch im Verhältnis 6 : 3 : 1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

**Beispiel B 4**: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60 - 90 % der Kontrolle), sowie teilweise eine Zunahme der Stengeldurchmesser auf.

**Beispiel B 5**: Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt. Die Verbindungen der Formel I bewirken eine Reduzierung des Neuzuwachses von etwa 10 bis 30 % im Vergleich zur unbehandelten Kontrolle.

**Patentansprüche** für die Vertragsstaaten: DE, GB, FR, CH, IT, NL, BE

1. N-Arylsulfonyl-N'-triazinyl- und -pyrimidinyl-harnstoffe der allgemeinen Formel I

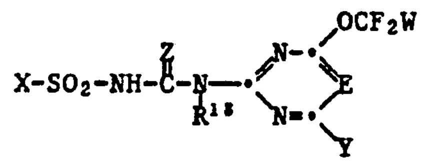

(I),

worin

X einen Rest der Formel 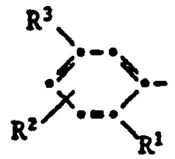 oder 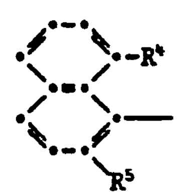 ,

Y $C_1$-$C_3$-Alkyl, $C_1$-$C_3$-Halogenalkyl, $C_1$-$C_3$-Alkoxy, $C_1$-$C_3$-Halogenalkoxy, $C_2$-$C_3$-Alkoxyalkyl, $C_1$-$C_3$-Alkylthio, Halogen oder $-NR^{16}R^{17}$,
Z Sauerstoff oder Schwefel,
E Stickstoff oder die Methinbrücke -CH=,
W Chlor oder Brom,
$R^1$ Wasserstoff, Halogen, Cyan, Nitro, $C_1$-$C_4$-Halogenalkyl, $C_1$-$C_4$-Alkyl, $C_1$-$C_4$-Alkoxy, $-CO-R^6$, $-NR^7R^8$, $-S(O)_m$-$C_1$-$C_4$-Alkyl oder $-SO_2R^9$,
$R^2$ Wasseretoff, Fluor, Chlor, Brom, Nitro, Trifluormethyl, $-NR^{20}R^{21}$, Methyl, Äthyl, Methoxy, Äthoxy oder $-S(O)_m$-$C_1$-$C_4$-Alkyl,
$R^3$ Wasserstoff, Fluor, Chlor, Brom, Amino, Nitro oder Methoxy,
$R^6$ Wasserstoff, $C_1$-$C_4$-Alkyl, $C_3$-$C_5$-Alkenyloxy, $C_3$-$C_5$-Alkinyloxy, $C_1$-$C_4$-Halogenalkyl, $C_1$-$C_5$-Alkylthio, Phenoxy, Benzyloxy, $-NR^{10}R^{11}$ oder gegebenenfalls durch 1 - 3 Halogenatome oder $C_1$-$C_3$-Alkoxy substituiertes $C_1$-$C_5$-Alkoxy,
$R^7$ Wasserstoff, Methoxy, Äthoxy, $C_1$-$C_4$-Alkyl oder $-CO-R^{12}$,
$R^8$ Wasserstoff oder $C_1$-$C_4$-Alkyl,
$R^9$ eine Cruppe $-O-R^{13}$ oder $-NR^{14}R^{15}$,
$R^{13}$ gegebenenfalls durch 1 - 3 Halogenatome substituiertes $C_1$-$C_4$-Alkyl, Phenyl oder Benzyl,
$R^{18}$ Wasserstoff, $C_1$-$C_3$-Alkyl oder $C_1$-$C_3$-Alkoxy und
m die Zahlen Null, eins oder zwei bedeuten,

wobei
$R^4$ die gleiche Bedeutung wie $R^2$;
$R^5$ die gleiche Bedeutung wie $R^1$;
$R^{10}$, $R^{11}$, $R^{14}$ und $R^{20}$ die gleiche Bedeutung wie $R^7$; und
$R^{12}$, $R^{15}$, $R^{16}$, $R^{17}$ und $R^{21}$ die gleiche Bedeutung wie $R^8$ haben;
sowie die Salzen dieser Verbindungen.

2. Verbindungen gemäss Anspruch 1, dadurch gekennzeichnet, dass X den gegebenenfalls substituierten Phenylrest bedeutet.

3. Verbindungen gemäss Anspruch 1, dadurch gekennzeichnet, dass Y einen Rest mit höchstens 2

Kohlenstoffatomen bedeutet.

4. Verbindungen gemäss Anspruch 1, dadurch gekennzeichnet, dass Z Sauerstoff bedeutet.

5. Verbindungen gemäss Anspruch 1, dadurch gekennzeichnet, dass E die Methinbrücke =CH- bedeutet.

6. Verbindungen gemäss Anspruch 1, dadurch gekennzeichnet, dass W Brom bedeutet.

7. Verbindungen gemäss Anspruch 1, dadurch gekennzeichnet, dass $R^3$ und $R^4$ Wasserstoff bedeuten.

8. Verbindungen gemäss Anspruch 1, dadurch gekennzeichnet, dass $R^{18}$ Wasserstoff oder Methyl bedeutet.

9. Verbindungen gemäss Anspruch 1, dadurch gekennzeichnet, dass
X den gegebenenfalls substituierten Phenylrest,
Y einen Rest mit höchstens 2 Kohlenstoffatomen,
Z Sauerstoff und
$R^3$ und $R^{18}$ Wasserstoff bedeuten.

10. Verbindungen gemäss Anspruch 9, dadurch gekennzeichnet, dass $R^2$ Wasserstoff bedeutet.

11. Verbindungen gemäss Anspruch 1, dadurch gekennzeichnet, dass
X den gegebenenfalls substituierten Phenylkern,
Y einen Rest mit höchstens 2 Kohlenstoffatomen,
Z Sauerstoff,
E eine Methingruppe =CH-,
W Brom und
$R^{18}$ Wasserstoff bedeuten, wobei
$R^1$ für Wasserstoff, Halogen, Nitro, $C_1$-$C_4$-Halogenalkyl, $C_1$-$C_4$-Alkoxy, $C_1$-$C_4$-Alkyl, $-S(O)_m-C_1-C_4$-Alkyl, $-SO_2-N(CH_3)_2$, $-SO_2-OCH_2CF_3$ oder $-CO-R^6$,
$R^2$ für Wasserstoff, Fluor, Chlor, Nitro, Amino, Trifluormethyl, Methyl, Methoxy, Äthoxy oder $-S(O)_m-C_1-C_4$-Alkyl,
$R^3$ für Wasserstoff, Fluor, Chlor, Brom, Amino, Nitro oder Methoxy,
$R^6$ für Wasserstoff, Methyl, $C_3$-$C_5$-Alkenyloxy, $C_3$-$C_5$-Alkinyloxy, $C_1$-$C_3$-Alkylthio, Dimethylamino, Methylamino, Amino oder gegebenenfalls durch 1 bis 3 Halogenatom, oder $C_1$-$C_3$-Alkoxy substituiertes $C_1$-$C_5$-Alkoxy und
m für die Zahlen Null, eins oder zwei stehen.

12. N-(2-Methoxycarbonylphenyl-sulfonyl)-N'-(4-difluorbrommethoxy-6-methylpyrimidin-2-yl)-harnstoff gemäss Anspruch 1.

13. N-(2-Methoxycarbonylphenyl-sulfonyl)-N'-(4-difluorbrommethoxy-6-methoxypyrimidin-2-yl)-harnstoff gemäss Anspruch 1.

14. N-(2-Nitrophenyl-sulfonyl)-N'-(4-difluorbrommethoxy-6-methylpyrimidin-2-yl)-harnstoff gemäss Anspruch 1.

15. Verfahren zur Herstellung der Verbindungen der Formel I gemäss Anspruch 1, dadurch gekennzeichnet, dass man ein Arylsulfonamid der Formel II

$$X - SO_2 - NH_2 \quad \text{(III)},$$

worin X die unter Formel I gegebene Bedeutung hat, in Gegenwart einer Base mit einem N-Triazinyl- oder N-Pyrimidinylcarbamat der Formel III

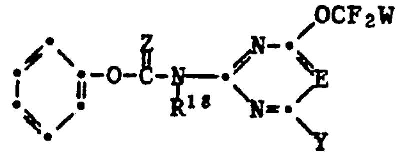
(III),

worin $R^{18}$
E, W, Y und Z die unter Formel I gegebene Bedeutung haben, umsetzt und gegebenenfalls in die Salze überführt.

16. Verfahren zur Herstellung der Verbindungen der Formel I gemäss Anspruch 1, dadurch gekennzeichnet, dass man ein Arylsulfonylisocyanat oder -isothiocyanat der Formel IV

$$X - SO_2 - N = C = Z \quad \text{(IV)}$$

worin X und Z die unter Formel I gegebene Bedeutung haben, gegebenenfalls in Gegenwart einer Base, mit einem Aminopyrimidin oder -triazin der Formel V

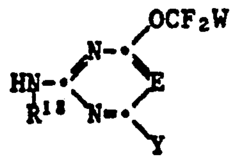

(V),

worin $R^{18}$, E, W und Y die unter Formel I gegebene Bedeutung haben, umsetzt und gegebenenfalls in die Salze überführt.

17. Verfahren zur Herstellung der Verbindungen der Formel I gemäss Anspruch 1, worin $R^{18}$ Wasserstoff bedeutet, dadurch gekennzeichnet, dass man ein Arylsulfonamid der oben angegebenen Formel II gegebenenfalls in Gegenwart einer Base mit einem Isocyanat oder Isothiocyanat der Formel VI

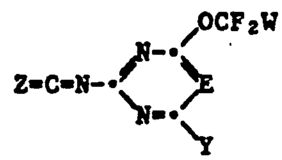

(VI),

worin E, W, Y und Z die unter Formel I gegebene Bedeutung haben, umsetzt und gegebenenfalls in die Salze überführt.

18. Verfahren zur Herstellung der Verbindungen der Formel I gemäss Anspruch 1, dadurch gekennzeichnet, dass man ein N-Arylsulfonylcarbamat der Formel VII

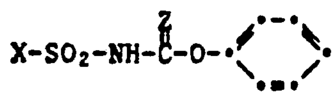

(VII),

worin X und Z die unter Formel I gegebene Bedeutung haben, mit einem Aminopyrimidin oder -triazin der oben angegebenen Formel V umsetzt und gegebenenfalls in die Salze überführt.

19. Verfahren zur Herstellung von Salzen der Formel I gemäss einem der Ansprüche 15 bis 18, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

20. Ein herbizides und den Pflanzenwuchs regulierendes Mittel, dadurch gekennzeichnet, dass es neben Träger- und/oder anderen Zuschlagstoffen als Wirkstoff mindestens einen N-Arylsulfonyl-N'-triazinyl- oder Pyrimidinyl-harnstoff der Formel I, Anspruch 1, enthält.

21. Die Verwendung der N-Arylsulfonyl-N'-triazinyl- und -pyrimidinyl-harnstoffe der Formel I, Anspruch 1, oder sie enthaltender Mittel zur Bekämpfung unerwünschten Pflanzenwachstums.

22. Die Verwendung von N-Arylsulfonyl-N'-triazinyl- und -pyrimidinyl-harnstoffen der Formel I, Anspruch 1, oder sie enthaltender Mittel zur Regulierung des Pflanzenwachstums.

23. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

24. Die Verwendung gemäss Anspruch 21 zur selektiven pre- oder postemergenten Bekämpfung von Unkräutern in Nutzpflanzenkulturen.

25. Die Verwendung gemäss Anspruch 24 zur Bekämpfung perennierender Unkräuter in Nutzpflanzenkulturen.

26. Die Verwendung gemäss Anspruch 22 zur Unterdrückung des Pflanzenwachstums über das 2-Blattstadium hinaus, dadurch gekennzeichnet, dass die Wirkstoffe preemergent angewendet werden.

27. Die Verwendung gemäss Anspruch 24 in Getreidekulturen.

28. Aminopyrimidine und Aminotriazine der Formel V

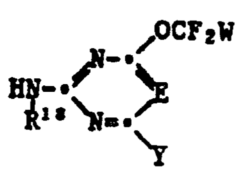

(V),

worin $R^{18}$, E, W und Y die unter Formel I, Anspruch 1, gegebenen Bedeutungen haben.

**Patentansprüche** für der Vertrafsstaat: Österreich

1. Ein herbizides und den Pflanzenwuchs regulierendes Mittel, dadurch gekennzeichnet, dass es neben Träger- und/oder anderen Zuschlagstoffen als Wirkstoff mindestens einen N-Arylsulfonyl-N'-triazinyl- und -pyrimidinyl-harnstoff der allgemeinen Formel I

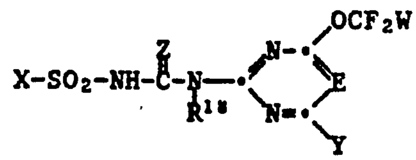 (I),

oder ein Salz dieser Verbindungen enthält, worin

X einen Rest der Formel 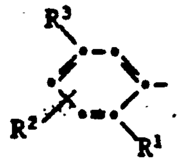 oder 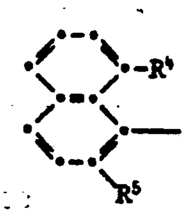 ,

Y $C_1$-$C_3$-Alkyl, $C_1$-$C_3$-Halogenalkyl, $C_1$-$C_3$-Alkoxy, $C_1$-$C_3$-Halogenalkoxy, $C_2$-$C_3$-Alkoxyalkyl, $C_1$-$C_3$-Alkylthio, Halogen oder $-NR^{16}R^{17}$,
Z Sauerstoff oder Schwefel,
E Stickstoff oder die Methinbrücke -CH=,
W Chlor oder Brom,
$R^1$ Wasserstoff, Halogen, Cyan, Nitro, $C_1$-$C_4$-Halogenalkyl, $C_1$-$C_4$-Alkyl, $C_1$-$C_4$-Alkoxy, $-CO-R^6$, $-NR^7R^8$, $-S(O)_m$-$C_1$-$C_4$-Alkyl oder $-SO_2R^9$,
$R^2$ Wasserstoff, Fluor, Chlor, Brom, Nitro, Trifluormethyl, $-NR^{20}R^{21}$, Methyl, Äthyl, Methoxy, Äthoxy oder $-S(O)_m$-$C_1$-$C_4$-Alkyl,
$R^3$ Wasserstoff, Fluor, Chlor, Brom, Amino, Nitro oder Methoxy,
$R^6$ Wasserstoff, $C_1$-$C_4$-Alkyl, $C_3$-$C_5$-Alkenyloxy, $C_3$-$C_5$-Alkinyloxy, $C_1$-$C_4$-Halogenalkyl, $C_1$-$C_5$-Alkylthio, Phenoxy, Benzyloxy, $-NR^{10}R^{11}$ oder gegebenenfalls durch 1 - 3 Halogenatome oder $C_1$-$C_3$-Alkoxy substituiertes $C_1$-$C_5$-Alkoxy,
$R^7$ Wasserstoff, Methoxy, Äthoxy, $C_1$-$C_4$-Alkyl oder $-CO-R^{12}$,
$R^8$ Wasserstoff oder $C_1$-$C_4$-Alkyl,
$R^9$ eine Gruppe $-O-R^{13}$ oder $-NR^{14}R^{15}$,
$R^{13}$ gegebenenfalls durch 1 - 3 Halogenatome substituiertes $C_1$-$C_4$-Alkyl, Phenyl oder Benzyl,
$R^{18}$ Wasserstoff, $C_1$-$C_3$-Alkyl oder $C_1$-$C_3$-Alkoxy und
m die Zahlen Null, eins oder zwei bedeuten,

wobei
$R^4$ die gleiche Bedeutung wie $R^2$;
$R^5$ die gleiche Bedeutung wie $R^1$;
$R^{10}$, $R^{11}$, $R^{14}$ und $R^{20}$ die gleiche Bedeutung wie $R^7$; und
$R^{12}$, $R^{15}$, $R^{16}$, $R^{17}$ und $R^{21}$ die gleiche Bedeutung wie $R^8$ haben.

2. Mittel gemäss Anspruch 1, dadurch gekennzeichnet, dass X den gegebenenfalls substituierten Phenylrest bedeutet.

3. Mittel gemäss Anspruch 1, dadurch gekennzeichnet, dass Y einen Rest mit höchstens 2 Kohlenstoffatomen bedeutet.

4. Mittel gemäss Anspruch 1, dadurch gekennzeichnet, dass Z Sauerstoff bedeutet.

5. Mittel gemäss Anspruch 1, dadurch gekennzeichnet, dass E die Methinbrücke =CH- bedeutet.

6. Mittel gemäss Anspruch 1, dadurch gekennzeichnet, dass W Brom bedeutet.

7. Mittel gemäss Anspruch 1, dadurch gekennzeichnet, dass $R^3$ und $R^4$ Wasserstoff bedeuten.

8. Mittel gemäss Anspruch 1, dadurch gekennzeichnet, dass $R^{18}$ Wasserstoff oder Methyl bedeutet.

9. Mittel gemäss Anspruch 1, dadurch gekennzeichnet, dass
X den gegebenenfalls substituierten Phenylrest,
Y einen Rest mit höchstens 2 Kohlenstoffatomen,
Z Sauerstoff und
$R^3$ und $R^{18}$ Wasserstoff bedeuten.

10. Mittel gemäss Anspruch 9, dadurch gekennzeichnet, dass $R^2$ Wasserstoff bedeutet.

11. Mittel gemäss Anspruch 1, dadurch gekennzeichnet, dass
X den gegebenenfalls substituierten Phenylkern,
Y einen Rest mit höchstens 2 Kohlenstoffatomen,
Z Sauerstoff,
E eine Methingruppe =CH-, W Brom und
$R^{18}$ Wasserstoff bedeuten, wobei
$R^1$ für Wasserstoff, Halogen, Nitro, $C_1$-$C_4$-Halogenalkyl, $C_1$-$C_4$-Alkoxy, $C_1$-$C_4$-Alkyl, $-S(O)_m-C_1$-$C_4$-Alkyl, $-SO_2-N(CH_3)_2$, $-SO_2-OCH_2CF_3$ oder $-CO-R^6$,
$R^2$ für Wasserstoff, Fluor, Chlor, Nitro, Amino, Trifluormethyl, Methyl, Methoxy, Äthoxy oder $-S(O)_m-C_1$-$C_4$-Alkyl,
$R^3$ für Wasserstoff, Fluor, Chlor, Brom, Amino, Nitro oder Methoxy,
$R^6$ für Wasserstoff, Methyl, $C_3$-$C_5$-Alkenyloxy, $C_3$-$C_5$-Alkinyloxy, $C_1$-$C_3$-Alkylthio, Dimethylamino, Methylamino, Amino oder gegebenenfalls durch 1 bis 3 Halogenatome oder $C_1$-$C_3$-Alkoxy substituiertes $C_1$-$C_5$-Alkoxy und
m für die Zahlen Null, eins oder zwei stehen.

12. Mittel gemäss Anspruch 1, dadurch gekennzeichnet, dass es als Wirkstoff N-(2-Methoxycarbonylphenyl-sulfonyl)-N'-(4-difluorbrommethoxy-6-methylpyrimidin-2-yl)-harnstoff gemäss Anspruch 1 enthält.

13. Mittel gemäss Anspruch 1, dadurch gekennzeichnet, dass es als Wirkstoff N-(2-Methoxycarbonylphenyl-sulfonyl)-N'-(4-difluorbrommethoxy-6-methoxypyrimidin-2-yl)-harnstoff gemäss Anspruch enthält.

14. Mittel gemäss Anspruch 1, dadurch gekennzeichnet, dass es als Wirkstoff N-(2-Nitrophenyl-sulfonyl)-N'-(4-difluorbrommethoxy-6-methylpyrimidin-2-yl)-harnstoff gemäss Anspruch 1 enthält.

15. Verfahren zur Herstellung der Verbindungen der Formel I gemäss Anspruch 1, dadurch gekennzeichnet, dass man ein Arylsulfonamid der Formel II

$$X-SO_2-NH_2 \qquad (II),$$

worin X die unter Formel I gegebene Bedeutung hat, in Gegenwart einer Base mit einem N-Triazinyl- oder N-Pyrimidinylcarbamat der Formel III

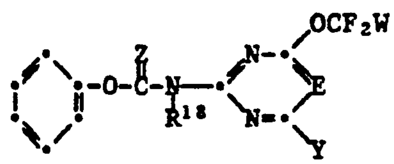
(III),

worin $R^{18}$, E, W, Y und Z die unter Formel I gegebene Bedeutung haben, umsetzt und gegebenenfalls in die Salze überführt.

16. Verfahren zur Herstellung der Verbindungen der Formel I gemäss Anspruch 1, dadurch gekennzeichnet, dass man ein Arylsulfonylisocyanat oder -isothiocyanat der Formel IV

$$X-SO_2-N=C=Z \qquad (IV)$$

worin X und Z die unter Formel I gegebene Bedeutung haben, gegebenenfalls in Gegenwart einer Base, mit einem Aminopyrimidin oder -triazin der Formel V

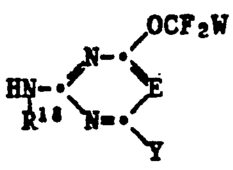
(V),

worin $R^{18}$, E, W und Y die unter Formel I gegebene Bedeutung haben, umsetzt und gegebenenfalls in die Salze überführt.

17. Verfahren zur Herstellung der Verbindungen der Formel I gemäss Anspruch 1, worin $R^{18}$ Wasserstoff bedeutet, dadurch gekennzeichnet, dass man ein Arylsulfonamid der oben angegebenen Formel II gegebenenfalls in Gegenwart einer Base mit einem Isocyanat oder Isothiocyanat der Formel VI

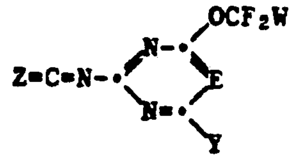

(VI),

worin E, W, Y und Z die unter Formel I gegebene Bedeutung haben, umsetzt und gegebenenfalls in die Salze überführt.

18. Verfahren zur Herstellung der Verbindungen der Formel I gemäss Anspruch 1, dadurch gekennzeichnet, dass man ein N-Arylsulfonylcarbamat der Formel VII

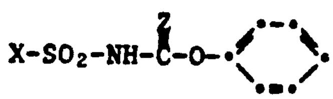

(VII),

worin X und Z die unter Formel I gegebene Bedeutung haben, mit einem Aminopyrimidin oder -triazin der oben angegebenen Formel V umsetzt und gegebenenfalls in die Salze überführt.

19. Verfahren zur Herstellung von Salzen der Formel I gemäss einem der Ansprüche 15 bis 18, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

20. Die Verwendung der N-Arylsulfonyl-N'-triazinyl- und -pyrimidinyl-harnstoffe der Formel I, Anspruch 1, oder sie enthaltender Mittel zur Bekämpfung unerwünschten Pflanzenwachstums.

21. Die Verwendung von N-Arylsulfonyl-N'-triazinyl- und -pyrimidinyl-harnstoffen der Formel I, Anspruch 1, oder sie enthaltender Mittel zur Regulierung des Pflanzenwachstums.

22. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

23. Die Verwendung gemäss Anspruch 20 zur selektiven pre- oder postemergenten Bekämpfung von Unkräutern in Nutzpflanzenkulturen.

24. Die Verwendung gemäss Anspruch 23 zur Bekämpfung perennierender Unkräuter in Nutzpflanzenkulturen.

25. Die Verwendung gemäss Anspruch 21 zur Unterdrückung des Pflanzenwachstums über das 2-Blattstadium hinaus, dadurch gekennzeichnet, dass die Wirkstoffe preemergent angewendet werden.

26. Die Verwendung gemäss Anspruch 23 in Getreidekulturen.

**Claims** for Contracting States: DE, GB, FR, CH, LI, IT, NL, BE.

1. N-arylsulfonyl-N'-triazinyl- and -pyrimidinyl-ureas of the general formula I

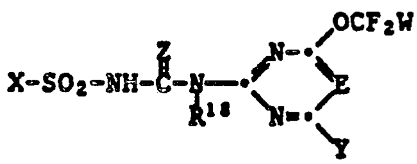

(I)

wherein

X is a radical of the formula

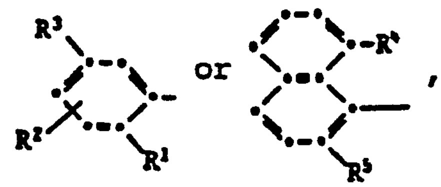

Y is $C_1$-$C_3$alkyl, $C_1$-$C_3$haloalkyl, $C_1$-$C_3$alkoxy, $C_1$-$C_3$-haloalkoxy, $C_2$-$C_3$alkoxyalkyl, $C_1$-$C_3$alkylthio, halogen or $-NR^{16}R^{17}$,

Z is oxygen or sulfur,

E is nitrogen or the methine bridge -CH=,

W is chlorine or bromine,

$R^1$ is hydrogen, halogen, cyano, nitro, $C_1$-$C_4$haloalkyl, $C_1$-$C_4$alkyl, $C_1$-$C_4$alkoxy, $-CO-R^6$, $-NR^7R^8$, $-S(O)_m$-$C_1$-$C_4$alkyl or $-SO_2R^9$,

$R^2$ is hydrogen, fluorine, chlorine, bromine, nitro, trifluoromethyl, $-NR^{20}R^{21}$, methyl, ethyl, methoxy, ethoxy or $-S(O)_m$-$C_1$-$C_4$alkyl,

$R^3$ is hydrogen, fluorine, chlorine, bromine, amino, nitro or methoxy,

$R^6$ is hydrogen, $C_1$-$C_4$alkyl, $C_3$-$C_5$alkenyloxy, $C_3$-$C_5$alkynyloxy, $C_1$-$C_4$haloalkyl, $C_1$-$C_5$alkylthio, phenoxy, benzyloxy, $-NR^{10}R^{11}$, or is $C_1$-$C_5$alkoxy which is unsubstituted or substituted by 1 to 3 halogen atoms or by $C_1$-$C_3$alkoxy,

$R^7$ is hydrogen, methoxy, ethoxy, $C_1$-$C_4$alkyl or $-CO-R^{12}$,

$R^8$ is hydrogen or $C_1$-$C_4$alkyl,

$R^9$ is an $-O-R^{13}$ or $-NR^{14}R^{15}$ group,

$R^{13}$ is $C_1$-$C_4$alkyl which is unsubstituted or substituted by 1 to 3 halogen atoms, or is phenyl or benzyl,

$R^{18}$ is hydrogen, $C_1$-$C_3$alkyl or $C_1$-$C_3$alkoxy and

m is 0, 1 or 2,

and

$R^4$ has the same meaning as $R^2$;

$R^5$ has the same meaning as $R^1$;

$R^{10}$, $R^{11}$ $R^{14}$ and $R^{20}$ have the same meaning as $R^7$; and

$R^{12}$, $R^{15}$, $R^{16}$, $R^{17}$ and $R^{21}$ have the same meaning as $R^8$;

and the salts of these compounds.

2. Compounds according to claim 1, wherein X is the unsubstituted or substituted phenyl radical.

3. Compounds according to claim 1, wherein Y is a radical containing not more than 2 carbon atoms.

4. Compounds according to claim 1, wherein Z is oxygen.

5. Compounds according to claim 1, wherein E is the methine bridge =CH-.

6. Compounds according to claim 1, wherein W is bromine.

7. Compounds according to claim 1, wherein $R^3$ and $R^4$ are hydrogen.

8. Compounds according to claim 1, wherein $R^{18}$ is hydrogen or methyl.

9. Compounds according to claim 1, wherein
X is the unsubstituted or substituted phenyl radical,
Y is a radical containing not more than 2 carbon atoms,
Z is oxygen, and
$R^3$ and $R^{18}$ are hydrogen.

10. Compounds according to claim 9, wherein $R^2$ is hydrogen.

11. Compounds according to claim 1, wherein
X is the unsubstituted or substituted phenyl nucleus,
Y is a radical containing not more than 2 carbon atoms,
Z is oxygen,
E is the methine group =CH-,
W is bromine and
$R^{18}$ is hydrogen, and
$R^1$ is hydrogen, halogen, nitro, $C_1$-$C_4$haloalkyl, $C_1$-$C_4$alkoxy, $C_1$-$C_4$alkyl, $-S(O)_m$-$C_1$-$C_4$alkyl, $-SO_2-N(CH_3)_2$, $-SO_2-OCH_2CF_3$ or $-CO-R^6$,
$R^2$ is hydrogen, fluorine, chlorine, nitro, amino, trifluoromethyl, methyl, methoxy, ethoxy or $-S(O)_m$-$C_1$-$C_4$alkyl,
$R^3$ is hydrogen, fluorine, chlorine, bromine, amino, nitro or methoxy, $R^6$ is hydrogen, methyl, $C_3$-$C_5$alkenyloxy, $C_3$-$C_5$alkynyloxy, $C_1$-$C_3$alkylthio, dimethylamino, methylamino, amino, or is $C_1$-$C_5$alkoxy which is unsubstituted or substituted by 1 to 3 halogen atoms or by $C_1$-$C_3$alkoxy, and
m is 0, 1 or 2.

12. N-(2-Methoxycarbonylphenylsulfonyl)-N'-(4-difluorobromomethoxy-6-methylpyrimidin-2-yl)urea according to claim 1.

13. N-(2-Methoxycarbonylphenylsulfonyl)-N'-(4-difluorobromomethoxy-6-methoxypyrimidin-2-yl)urea according to claim 1.

14. N-(2-Nitrophenylsulfonyl)-N'-(4-difluorobromomethoxy-6-methylpyrimidin-2-yl)urea according to claim 1.

15. A process for the preparation of the compounds of formula I according to claim 1, which comprises reacting an arylsulfonamide of formula II

$$X - SO_2 - NH_2 \qquad (II),$$

wherein X is as defined for formula I, with an N-triazinylcarbamate or N-pyrimidinylcarbamate of formula III

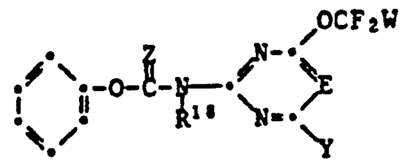

(III),

wherein $R^{18}$, E, W, Y and Z are as defined for formula I, in the presence of a base, and, if desired, converting the resultant compound of formula I into a salt.

16. A process for the preparation of the compounds of formula I according to claim 1, which comprises reacting an arylsulfonylisocyanate or arylsulfonylisothiocyanate of formula IV

$$X - SO_2 - N = C = Z \qquad (IV),$$

wherein X and Z are as defined for formula I, with an aminopyrimidine or aminotriazine of formula V

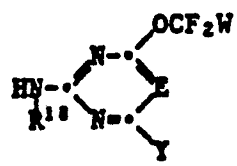

(V),

wherein $R^{18}$, E, W and Y are as defined for formula I, in the absence or presence of a base, and, if desired, converting the resultant compound of formula I into a salt.

17. A process for the preparation of the compounds of formula I according to claim 1, wherein $R^{18}$ is hydrogen, which comprises reacting an arylsulfonamide of the above formula II with an isocyanate or isothiocyanate of formula VI

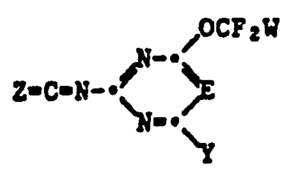

(VI),

wherein E, W, Y and Z are as defined for formula I, in the absence or presence of a base, and, if desired, converting the resultant compound of formula I into a salt.

18. A process for the preparation of the compounds of formula I according to claim 1, which comprises reacting an N-arylsulfonylcarbamate of formula VII

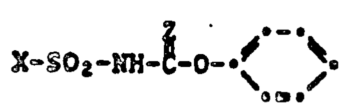

(VII),

wherein X and Z are as defined for formula I, with an aminopyrimidine or aminotriazine of the above formula V, and, if desired, converting the resultant compound of formula I into a salt.

19. A process for the preparation of salts of formula I according to any one of claims 15 to 18, which comprises reacting a sulfonylurea of formula I with an amine, an alkali metal hydroxide or an alkaline earth metal hydroxide or with a quaternary ammonium base.

20. A herbicidal and plant growth regulating composition, which contains, as active ingredient, at least one N-arylsulfonyl-N'-triazinylurea or N-arylsulfonyl-N'-pyrimidinylurea of formula I, claim 1, together with carriers and/or other adjuvants.

21. The use of N-arylsulfonyl-N'-triazinyl- and -pyrimidinyl-ureas of formula I, claim 1, or of compositions

containing them, for controlling undesired plant growth.

22. The use of N-arylsulfonyl-N'-triazinyl- and -pyrimidinyl-ureas of formula I, claim 1, or of compositions containing them, for regulating plant growth.

23. The use of N-phenylsulfonyl-N'-triazinyl- or -pyrimidinyl-ureas of formula I, claim 1, or of compositions containing them, for regulating the growth of cultivated plants for the purpose of increasing yield.

24. The use according to claim 21 for selectively controlling weeds pre- or post-emergence in crops of useful plants.

25. The use according to claim 24 for controlling perennial weeds in crops of useful plants.

26. The use according to claim 22 for suppressing plant growth beyond the 2-leaf stage, which comprises applying the active ingredients pre-emergence.

27. The use according to claim 24 in cereal crops.

28. Aminopyrimidines and aminotriazines of formula V

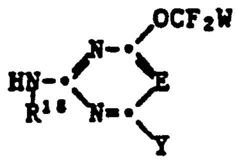

(V),

wherein $R^{18}$, E, W and Y are as defined for formula I, claim 1.

**Claims** (for Contracting State: AT)

1. A herbicidal and plant growth regulating composition which contains as active ingredient, together with carriers and/or other adjuvants, at least one N-arylsulfonyl-N'-triazinyl- or -pyrimidinyl-urea of the general formula I

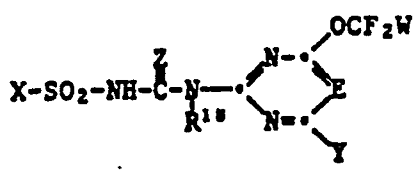

(I),

or a salt of these compounds,
wherein

X is a radical of the formula

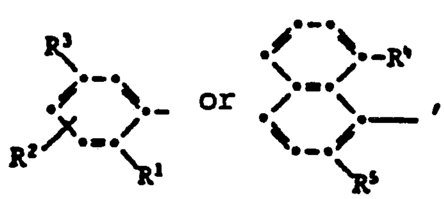

Y is $C_1$-$C_3$alkyl, $C_1$-$C_3$haloalkyl, $C_1$-$C_3$alkoxy, $C_1$-$C_3$-haloalkoxy, $C_2$-$C_3$alkoxyalkyl, $C_1$-$C_3$alkylthio, halogen or $-NR^{16}R^{17}$,

Z is oxygen or sulfur,

E is nitrogen or the methine bridge -CH=,

W is chlorine or bromine,

$R^1$ is hydrogen, halogen, cyano, nitro, $C_1$-$C_4$haloalkyl, $C_1$-$C_4$alkyl, $C_1$-$C_4$alkoxy, $-CO-R^6$, $-NR^7R^8$, $-S(O)_m-C_1-C_4$alkyl or $-SO_2R^9$,

$R^2$ is hydrogen, fluorine, chlorine, bromine, nitro, trifluoromethyl, $-NR^{20}R^{21}$, methyl, ethyl, methoxy, ethoxy or $-S(O)_m-C_1-C_4$alkyl,

$R^3$ is hydrogen, fluorine, chlorine, bromine, amino, nitro or methoxy,

$R^6$ is hydrogen, $C_1$-$C_4$alkyl, $C_3$-$C_5$alkenyloxy, $C_3$-$C_5$alkynyloxy, $C_1$-$C_4$haloalkyl, $C_1$-$C_5$alkylthio, phenoxy, benzyloxy, $-NR^{10}R^{11}$, or is $C_1$-$C_5$alkoxy which is unsubstituted or substituted by 1 to 3 halogen atoms or by $C_1$-$C_3$alkoxy,

$R^7$ is hydrogen, methoxy, ethoxy, $C_1$-$C_4$alkyl or $-CO-R^{12}$,

$R^8$ is hydrogen or $C_1$-$C_4$alkyl,
$R^9$ is an -O-$R^{13}$ or -$NR^{14}R^{15}$ group,
$R^{13}$ is $C_1$-$C_4$alkyl which is unsubstituted or substituted by 1 to 3 halogen atoms, or is phenyl or benzyl,
$R^{18}$ is hydrogen, $C_1$-$C_3$alkyl or $C_1$-$C_3$alkoxy and
m is 0, 1 or 2,
and
$R^4$ has the same meaning as $R^2$;
$R^5$ has the same meaning as $R^1$;
$R^{10}$, $R^{11}$ $R^{14}$ and $R^{20}$ have the same meaning as $R^7$; and
$R^{12}$, $R^{15}$, $R^{16}$, $R^{17}$ and $R^{21}$ have the same meaning as $R^8$.

2. A composition according to claim 1, wherein X is the unsubstituted or substituted phenyl radical.
3. A composition according to claim 1, wherein Y is a radical containing not more than 2 carbon atoms.
4. A composition according to claim 1, wherein Z is oxygen.
5. A composition according to claim 1, wherein E is the methine bridge =CH-.
6. A composition according to claim 1, wherein W is bromine.
7. A composition according to claim 1, wherein $R^3$ and $R^4$ are hydrogen.
8. A composition according to claim 1, wherein $R^{18}$ is hydrogen or methyl.
9. A composition according to claim 1, wherein
X is the unsubstituted or substituted phenyl radical,
Y is a radical containing not more than 2 carbon atoms,
Z is oxygen and $R^3$ and $R^{18}$ are hydrogen.
10. A composition according to claim 9, wherein $R^2$ is hydrogen.
11. A composition according to claim 1, wherein
X is the unsubstituted or substituted phenyl nucleus,
Y is a radical containing not more than 2 carbon atoms,
Z is oxygen,
E is the methine group =CH-,
W is bromine and
$R^{18}$ is hydrogen, and
$R^1$ is hydrogen, halogen, nitro, $C_1$-$C_4$haloalkyl, $C_1$-$C_4$alkoxy, $C_1$-$C_4$alkyl, -$S(O)_m$-$C_1$-$C_4$alkyl, -$SO_2$-$N(CH_3)_2$, -$SO_2$-$OCH_2CF_3$ or -CO-$R^6$,
$R^2$ is hydrogen, fluorine, chlorine, nitro, amino, trifluoromethyl, methyl, methoxy, ethoxy or -$S(O)_m$-$C_1$-$C_4$alkyl,
$R^3$ is hydrogen, fluorine, chlorine, bromine, amino, nitro or methoxy,
$R^6$ is hydrogen, methyl, $C_3$-$C_5$alkenyloxy, $C_3$-$C_5$alkynyloxy, $C_1$-$C_3$alkylthio, dimethylamino, methylamino, amino, or is $C_1$-$C_5$alkoxy which is unsubstituted or substituted by 1 to 3 halogen atoms or by $C_1$-$C_3$alkoxy, and
m is 0, 1 or 2.
12. A composition according to claim 1, which contains as active ingredient N-(2-methoxycarbonylphenylsulfonyl)-N'-(4-difluorobromomethoxy-6-methylpyrimidin-2-yl)urea according to claim 1.
13. A composition according to claim 1, which contains as active ingredient N-(2-methoxycarbonylphenylsulfonyl)-N'-(4-difluorobromomethoxy-6-methoxypyrimidin-2-yl)urea according to claim 1.
14. A composition according to claim 1, which contains as active ingredient N-(2-nitrophenylsulfonyl)-N'-(4-difluorobromomethoxy-6-methylpyrimidin-2-yl)urea according to claim 1.
15. A process for the preparation of the compounds of formula I according to claim 1, which comprises reacting an arylsulfonamide of formula II

$$X - SO_2 - NH_2 \qquad (II),$$

wherein X is as defined for formula I, with an N-triazinylcarbamate or N-pyrimidinylcarbamate of formula III

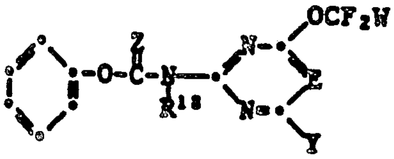

(III),

wherein $R^{18}$, E, W, Y and Z are as defined for formula I, in the presence of a base, and, if desired, converting the resultant compound of formula I into a salt.
16. A process for the preparation of the compounds of formula I according to claim 1, which comprises reacting an arylsulfonylisocyanate or arylsulfonylisothiocyanate of formula IV

$$X - SO_2 - N = C = Z \qquad (IV),$$

wherein X and Z are as defined for formula I, with an aminopyrimidine or aminotriazine of formula V

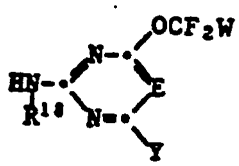

(V),

wherein $R^{18}$, E, W and Y are as defined for formula I, in the absence or presence of a base, and, if desired, converting the resultant compound of formula I into a salt.

17. A process for the preparation of the compounds of formula I according to claim 1, wherein $R^{18}$ is hydrogen, which comprises reacting an arylsulfonamide of the above formula II with an isocyanate or isothiocyanate of formula VI

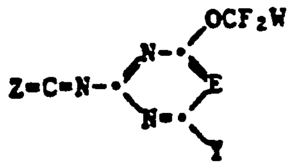

(VI),

wherein E, W, Y and Z are as defined for formula I, in the absence or presence of a base, and, if desired, converting the resultant compound of formula I into a salt.

18. A process for the preparation of the compounds of formula I according to claim 1, which comprises reacting an N-arylsulfonylcarbamate of formula VII

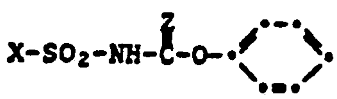

(VII),

wherein X and Z are as defined for formula I, with an aminopyrimidine or aminotriazine of the above formula V, and, if desired, converting the resultant compound of formula I into a salt.

19. A process for the preparation of salts of formula I according to any one of claims 15 to 18, which comprises reacting a sulfonylurea of formula I with an amine, an alkali metal hydroxide or an alkaline earth metal hydroxide or with a quaternary ammonium base.

20. The use of N-arylsulfonyl-N'-triazinyl- and -pyrimidinyl-ureas of formula I, claim 1, or of compositions containing them, for controlling undesired plant growth.

21. The use of N-arylsulfonyl-N'-triazinyl- and -pyrimidinyl-ureas of formula I, claim 1, or of compositions containing them, for regulating plant growth.

22. The use of N-phenylsulfonyl-N'-triazinyl- or -pyrimidinyl-ureas of formula I, claim 1, or of compositions containing them, for regulating the growth of cultivated plants for the purpose of increasing yield.

23. The use acording to claim 20 for selectively controlling weeds pre- or post-emergence in crops of useful plants.

24. The use according to claim 23 for controlling perennial weeds in crops of useful plants.

25. The use according to claim 21 for suppressing plant growth beyond the 2-leaf stage, which comprises applying the active ingredients pre-emergence.

26. The use according to claim 23 in cereal crops.

**Revendications** pour les Etats contractants DE, GB, FR, CH, LI, IT, NL, BE

1. N-arylsulfonyl-N'-triazinyl- et pyrimidinylurées de formule générale I

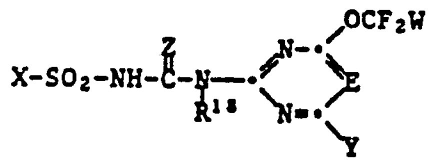 (I),

dans laquelle

X représente un groupe de formule

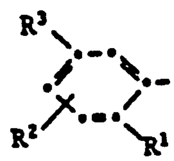 ou 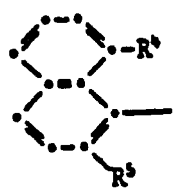 ,

Y représente un groupe alkyle en $C_1$-$C_3$, halogénoalkyle en $C_1$-$C_3$, alcoxy en $C_1$-$C_3$, halogénoalcoxy en $C_1$-$C_3$, alcoxyalkyle en $C_2$-$C_3$, alkylthio en $C_1$-$C_3$, un halogène ou un groupe $-NR^{16}R^{17}$,
Z représente l'oxygène ou le soufre,
E représente l'azote ou le pont méthine -CH=,
W représente le chlore ou le brome,
$R^1$ représente l'hydrogène, un halogène, un groupe cyano, nitro, halogénoalkyle en $C_1$-$C_4$, alkyle en $C_1$-$C_4$, alcoxy en $C_1$-$C_4$, $-CO-R^6$, $-NR^7R^8$, $-S(O)_m$-alkyle en $C_1$-$C_4$ ou $-SO_2R^9$,
$R^2$ représente l'hydrogène, le fluor, le chlore, le brome, un groupe nitro, trifluorométhyle, $-NR^{20}R^{21}$, méthyle, éthyle, méthoxy, éthoxy ou $-S(O)_m$-alkyle en $C_1$-$C_4$,
$R^3$ représente l'hydrogène, le fluor, le chlore, le brome, un groupe amino, nitro ou méthoxy,
$R^6$ représente l'hydrogène, un groupe alkyle en $C_1$-$C_4$, alcényloxy en $C_3$-$C_5$, alcynyloxy en $C_3$-$C_5$, halogénoalkyle en $C_1$-$C_4$, alkylthio en $C_1$-$C_5$, phénoxy, benzyloxy, $-NR^{10}R^{11}$ ou alcoxy en $C_1$-$C_5$ éventuellement substitué par 1 à 3 atomes d'halogènes ou groupes alcoxy en $C_1$-$C_3$,
$R^7$ représente l'hydrogène, un groupe méthoxy, éthoxy, alkyle en $C_1$-$C_4$ ou $-CO-R^{12}$,
$R^8$ représente l'hydrogène ou un groupe alkyle en $C_1$-$C_4$,
$R^9$ représente un groupe $-O-R^{13}$ ou $-NR^{14}R^{15}$,
$R^{13}$ représente un groupe alkyle en $C_1$-$C_4$, phényle ou benzyle, éventuellement substitué par 1 à 3 atomes d'halogènes,
$R^{18}$ représente l'hydrogène, un groupe alkyle en $C_1$-$C_3$ ou alcoxy en $C_1$-$C_3$, et
m est égal à 0, 1 ou 2,
et
$R^4$ a les mêmes significations que $R^2$;
$R^5$ a les mêmes significations que $R^1$;
$R^{10}$, $R^{11}$, $R^{14}$ et $R^{20}$ ont les mêmes significations que $R^7$; et
$R^{12}$, $R^{15}$, $R^{16}$, $R^{17}$ et $R^{21}$ ont les mêmes significations que $R^8$;
et les sels de ces composés.

2. Composés selon la revendication 1, caractérisés en ce que X représente le groupe phényle éventuellement substitué.

3. Composés selon la revendication 1, caractérisés en ce que Y représente un groupe à 2 atomes de carbone au maximum.

4. Composés selon la revendication 1, caractérisés en ce que Z représente l'oxygène.

5. Composés selon la revendication 1, caractérisés en ce que E représente le pont méthine =CH-.

6. Composés selon la revendication 1, caractérisés en ce que W représente le brome.

7. Composés selon la revendication 1, caractérisés en ce que $R^3$ et $R^4$ représentent l'hydrogène.

8. Composés selon la revendication 1, caractérisés en ce que $R^{18}$ représente l'hydrogène ou un groupe méthyle.

9. Composés selon la revendication 1, caractérisés en ce que
X représente le groupe phényle éventuellement substitué,
Y un groupe à 2 atomes de carbone au maximum,
Z l'oxygène et $R^3$ et $R^{18}$ l'hydrogène.

10. Composés selon la revendication 9, caractérisés en ce que $R^2$ représente l'hydrogène.

11. Composés selon la revendication 1, caractérisés en ce que
X représente le noyau phényle éventuellement substitué,
Y un groupe à 2 atomes de carbone au maximum,

Z l'oxygène,
E un groupe méthine =CH-,
W le brome et
$R^{18}$ l'hydrogène, et

$R^1$ représente l'hydrogène, un halogène, un groupe nitro, halogénoalkyle en $C_1$-$C_4$, alcoxy en $C_1$-$C_4$, alkyle en $C_1$-$C_4$, -$S(O)_m$-alkyle en $C_1$-$C_4$, -$SO_2$-$N(CH_3)_2$, -$SO_2$-$OCH_2CF_3$ ou -CO-$R^6$,

$R^2$ représente l'hydrogène, le fluor, le chlore, un groupe nitro, amino, trifluorométhyle, méthyle, méthoxy, éthoxy ou $S(O)_m$-alkyle en $C_1$-$C_4$,

$R^3$ représente l'hydrogène, le fluor, le chlore, le brome, un groupe amino, nitro ou méthoxy,

$R^6$ représente l'hydrogène, un groupe méthyle, alcényloxy en $C_3$-$C_5$, alcynyloxy en $C_3$-$C_5$, alkylthio en $C_1$-$C_3$, diméthylamino, méthylamino, amino ou alcoxy en $C_1$-$C_5$ éventuellement substitué par 1 à 3 atomes d'halogène ou groupes alcoxy en $C_1$-$C_3$, et

m est égal à 0, 1 ou 2.

12. La N-(2-méthoxycarbonylphényl-sulfonyl)-N'-(4-difluorobromométhoxy-6-méthylpyrimidine-2-yl)-urée selon la revendication 1.

13. La N-(2-méthoxycarbonylphényl-sulfonyl)-N'-(4-difluorobromométhoxy-6-méthoxypyrimidine-2-yl)-urée selon la revendication 1.

14. La N-(2-nitrophényl-sulfonyl)-N'-(4-difluorobromométhoxy-6-méthylpyrimidine-2-yl)-urée selon la revendication 1.

15. Procédé de préparation des composés de formule I selon la revendication 1, caractérisé en ce que l'on fait réagir un arylsulfonamide de formule II

$$X - SO_2 - NH_2 \qquad (II)$$

dans laquelle X a les significations indiquées en référence à la formule I, en présence d'une base, avec un N-triazinyl- ou N-pyrimidinylcarbamate de formule III

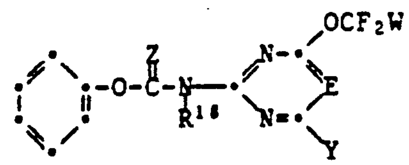

(III),

dans laquelle $R^{18}$, E, W, Y et Z ont les significations indiquées en référence à la formule I, et le cas échéant on convertit en les sels.

16. Procédé de préparation des composés de formule I selon la revendication 1, caractérisé en ce que l'on fait réagir un arylsulfonylisocyanate ou -isothiocyanate de formule IV

$$X - SO_2 - N = C = Z \qquad (IV)$$

dans laquelle X et Z ont les significations indiquées en référence à la formule I, éventuellement en présence d'une base, avec une aminopyrimidine ou -triazine de formule V

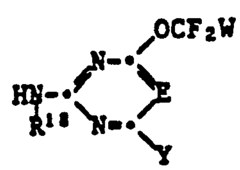

(V),

dans laquelle $R^{18}$, E, W et Y ont les significations indiquées en référence à la formule I, et le cas échéant on convertit en les sels.

17. Procédé de préparation des composés de formule I selon la revendication 1 dans lesquels $R^{18}$ représente l'hydrogène, caractérisé en ce que l'on fait réagir un arylsulfonamide de formule II ci-dessus, éventuellement en présence d'une base, avec un isocyanate ou isothiocyanate de formule VI

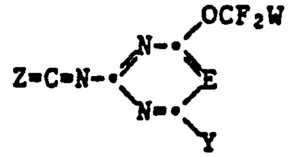

(VI),

dans laquelle E, W, Y et Z ont les significations indiquées en référence à la formule I, et le cas échéant on convertit en les sels.

18. Procédé de préparation des composés de formule I selon la revendication 1, caractérisé en ce que l'on fait réagir un N-arylsulfonylcarbamate de formule VII

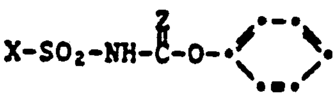

(VII),

dans laquelle X et Z ont les significations indiquées en référence à la formule I, avec un aminopyrimidine ou -triazine de formule V ci-dessus, et le cas échéant on convertit en les sels.

19. Procédé de préparation des sels de formule I selon l'une des revendications 15 à 18, caractérisé en ce que l'on fait réagir une sulfonylurée de formule I avec une amine, un hydroxyde de métal alcalin ou alcalinoterreux ou une base d'ammonium quaternaire.

20. Un produit herbicide et régulateur de la croissance des végétaux, caractérisé en ce qu'il contient en tant que substance active, avec des véhicules et/ou d'autres additifs, au moins une N-arylsulfonyl-N'-triazinyl- ou -pyrimidinyl-urée de formule I, revendication 1.

21. L'utilisation des N-arylsulfonyl-N'-triazinyl- et -pyrimidinyl-urées de formule I, revendication 1, et de produits en contenant dans la lutte contre les croissances des végétaux indésirables.

22. L'utilisation des N-arylsulfonyl-N'-triazinyl- et -pyrimidinyl-urées de formule I, revendication 1, ou de produits en contenant pour la régulation de la croissance des végétaux.

23. L'utilisation des N-phénylsulfonyl-N'-triazinyl- ou -pyrimidinyl-urées de formule I, revendication 1, ou de produits en contenant pour la régulation de la croissance des végétaux cultivés en vue d'une augmentation du rendement.

24. L'utilisation selon la revendication 21 pour la lutte sélective en pré-levée ou en post-levée contre les mauvaises herbes dans les cultures de végétaux utiles.

25. L'utilisation selon la revendication 24, pour la lutte contre les mauvaises herbes pérennes dans les cultures de végétaux utiles.

26. L'utilisation selon la revendication 22, pour l'inhibition de la croissance des végétaux au-delà du stade 2 feuilles, caractérisée en ce qu'on applique les substances actives en pré-levée.

27. L'utilisation selon la revendication 24, dans les cultures de céréales.

26. Aminopyrimidines et aminotriazines de formule V

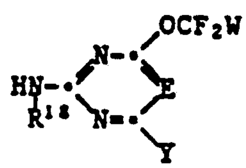

(V),

dans laquelle $R^{18}$, E, W et Y ont les significations indiquées en référence à la formule I, revendication 1.

**Revendications** pour l'Etat contractant AT

1. Un produit herbicide et régulateur de la croissance des végétaux, caractérisé en ce qu'il contient en tant que substance active, avec des véhicules et/ou d'autres additifs, au moins une N-arylsulfonyl-N'-triazinyl- et -pyrimidinyl-urée de formule générale I

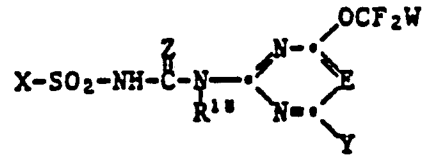 (I),

ou un sel d'un tel composé, dans lesquels

X représente un groupe de formule

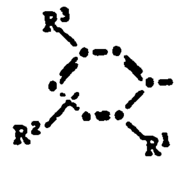 ou 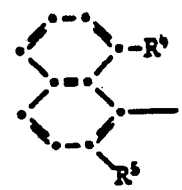 ,

Y représente un groupe alkyle en $C_1$-$C_3$, halogénoalkyle en $C_1$-$C_3$, alcoxy en $C_1$-$C_3$, halogénoalcoxy en $C_1$-$C_3$, alcoxyalkyle en $C_2$-$C_3$, alkylthio en $C_1$-$C_3$, un halogène ou un groupe $-NR^{16}R^{17}$,
Z représente l'oxygène ou le soufre,
E représente l'azote ou le pont méthine -CH=,
W représente le chlore ou le brome,
$R^1$ représente l'hydrogène, un halogène, un groupe cyano, nitro, halogénoalkyle en $C_1$-$C_4$, alkyle en $C_1$-$C_4$, alcoxy en $C_1$-$C_4$, $-CO-R^6$, $-NR^7R^8$, $-S(O)_m$-alkyle en $C_1$-$C_4$ ou $-SO_2R^9$,
$R^2$ représente l'hydrogène, le fluor, le chlore, le brome, un groupe nitro, trifluorométhyle, $-NR^{20}R^{21}$, méthyle, éthyle, méthoxy, éthoxy ou $-S(O)_m$-alkyle en $C_1$-$C_4$,
$R^3$ représente l'hydrogène, le fluor, le chlore, le brome, un groupe amino, nitro ou méthoxy,
$R^6$ représente l'hydrogène un groupe alkyle en $C_1$-$C_4$, alcényloxy en $C_3$-$C_5$, alcynyloxy en $C_3$-$C_5$, halogénoalkyle en $C_1$-$C_4$, alkylthio en $C_1$-$C_5$, phénoxy, benzyloxy, $-NR^{10}R^{11}$, ou un groupe alcoxy en $C_1$-$C_5$ éventuellement substitué par 1 à 3 atomes d'halogènes ou groupes alcoxy en $C_1$-$C_3$,
$R^7$ représente l'hydrogène, un groupe méthoxy, éthoxy, alkyle en $C_1$-$C_4$ ou $-CO-R^{12}$,
$R^8$ représente l'hydrogène ou un groupe alkyle en $C_1$-$C_4$,
$R^9$ représente un groupe $-O-R^{13}$ ou $-NR^{14}R^{15}$,
$R^{13}$ représente un groupe alkyle en $C_1$-$C_4$, phényle ou benzyle, éventuellement substitué par 1 à 3 atomes d'halogènes,
$R^{18}$ représente l'hydrogène, un groupe alkyle en $C_1$-$C_3$ ou alcoxy en $C_1$-$C_3$, et
m est égal à 0, 1 ou 2,
et
$R^4$ a les mêmes significations que $R^2$;
$R^5$ a les mêmes significations que $R^1$;
$R^{10}$, $R^{11}$, $R^{14}$ et $R^{20}$ ont les mêmes singifications que $R^7$;
$R^{12}$, $R^{15}$, $R^{16}$, $R^{17}$ et $R^{21}$ ont les mêmes significations que $R^8$.

2. Produit selon la revendication 1, caractérisé en ce que X représente le groupe phényle éventuellement substitué.

3. Produit selon la revendication 1, caractérisé en ce que Y représente un groupe à 2 atomes de carbone au maximum.

4. Produit selon la revendication 1, caractérisé en ce que Z représente l'oxygène.

5. Produit selon la revendication 1, caractérisé en ce que E représente le pont méthine =CH-.

6. Produit selon la revendication 1, caractérisé en ce que W représente le brome.

7. Produit selon la revendication 1, caractérisé en ce que $R^3$ et $R^4$ représentent l'hydrogène.

8. Produit selon la revendication 1, caractérisé en ce que $R^{18}$ représente l'hydrogène ou un groupe méthyle.

9. Produit selon la revendication 1, caractérisé en ce que
X représente le groupe phényle éventuellement substitué,
Y représente un groupe à 2 atomes de carbone au maximum,
Z représente l'oxygène et
$R^3$ et $R^{18}$ représententl'hydrogène.

10. Produit selon la revendication 9, caractérisé en ce que $R^2$ représente l'hydrogène.

11. Produit selon la revendication 1, caractérisé en ce que
X représente le noyau phényle éventuellement substitué,
Y représente un groupe à 2 atomes de carbone au maximum,
Z représente l'oxygène,

E représente un pont méthine =CH-,
W représente le brome et
$R^{18}$ l'hydrogène, et

$R^1$ représente l'hydrogène, un halogène, un groupe nitro, halogénoalkyle en $C_1$-$C_4$, alcoxy en $C_1$-$C_4$, alkyle en $C_1$-$C_4$, -$S(O)_m$-alkyle en $C_1$-$C_4$, -$SO_2$-$N(CH_3)_2$, -$SO_2$-$OCH_2CF_3$ ou -CO-$R^6$,

$R^2$ représente l'hydrogène, le fluor, le chlore, un groupe nitro, amino, trifluorométhyle, méthyle, méthoxy, éthoxy ou -$S(O)_m$-alkyle en $C_1$-$C_4$,

$R^3$ représente l'hydrogène, le fluor, le chlore, le brome, un groupe amino, nitro ou méthoxy,

$R^6$ représente l'hydrogène, un groupe méthyle, alcényloxy en $C_3$-$C_5$, alcynyloxy en $C_3$-$C_5$, alkylthio en $C_1$-$C_3$, diméthylamino, méthylamino, amino ou alcoxy en $C_1$-$C_5$ éventuellement substitué par 1 à 3 atomes d'halogènes ou groupes alcoxy en $C_1$-$C_3$, et

m est égal à 0, 1 ou 2.

12. Produit selon la revendication 1, caractérisé en ce qu'il contient en tant que substance active la N-(2-méthoxycarbonylphényl-sulfonyl)-N'-(4-difluorobromométhoxy-6-méthylpyrimidine-2-yl)-urée selon la revendication 1.

13. Produit selon la revendication 1, caractérisé en ce qu'il contient en tant que substance active la N-(2-méthoxycarbonylphényl-sulfonyl)-N'-(4-difluorobromométhoxy-6-méthoxypyrimidine-2-yl)-urée selon la revendication 1.

14. Produit selon la revendication 1, caractérisé en ce qu'il contient en tant que substance active la N-(2-nitrophényl-sulfonyl)-N'-(4-difluorobromométhoxy-6-méthylpyrimidine-2-yl)-urée selon la revendication 1.

15. Procédé de préparation des composés de formule I selon la revendication 1, caractérisé en ce que l'on fait réagir un arylsulfonamide de formule II

$$X - SO_2 - NH_2 \qquad (II)$$

dans laquelle X a les significations indiquées en référence à la formule I, en présence d'une base, avec un N-triazinyl- ou N-pyrimidinylcarbamate de formule III

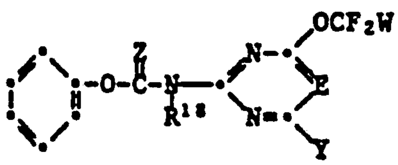

(III),

dans laquelle $R^{18}$, E, W, Y et Z ont les significations indiquées en référence à la formule I, et le cas échéant on convertit en les sels.

16. Procédé de préparation des composés de formule I selon la revendication 1, caractérisé en ce que l'on fait réagir un arylsulfonylisocyanate ou -isothiocyanate de formule IV

$$X - SO_2 - N = C = Z \qquad (IV)$$

dans laquelle X et Z ont les significations indiquées en référence à la formule I, éventuellement en présence d'une base, avec une aminopyrimidine ou -triazine de formule V

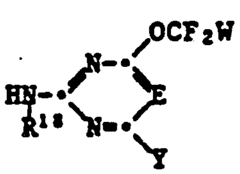

(V),

dans laquelle $R^{18}$, E, W et Y ont les significations indiquées en référence à la formule I, et le cas échéant on convertit en les sels.

17. Procédé de préparation des composés de formule I selon la revendication 1, dans lesquels $R^{18}$ représente l'hydrogène, caractérisé en ce que l'on fait réagir un arylsulfonamide de formule II ci-dessus, éventuellement en présence d'une base, avec un isocyanate ou isothiocyanate de formule VI

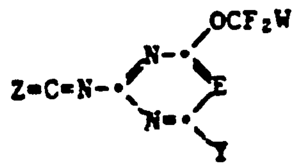

(VI),

dans laquelle E, W, Y et Z ont les significations indiquées en référence à la formule I, et le cas échéant on convertit en les sels.

18. Procédé de préparation des composés de formule I selon la revendication 1, caractérisé en ce que l'on fait réagir un N-arylsulfonylcarbamate de formule VII

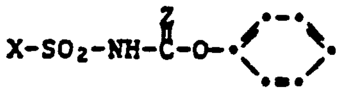

(VII),

dans laquelle X et Z ont les significations indiquées en référence à la formule I, avec une aminopyrimidine ou -triazine de formule V ci-dessus, et le cas échéant on convertit en les sels.

19. Procédé de préparation des sels de formule I selon l'une des revendications 15 à 18, caractérisé en ce que l'on fait réagir une sulfonylurée de formule I avec une amine, un hydroxyde de métal alcalin ou alcalinoterreux ou une base d'ammonium quaternaire.

20. L'utilisation des N-arylsulfonyl-N'-triazinyl- et -pyrimidinyl-urées de formule I, revendication 1, ou de produits en contenant dans la lutte contre les croissances de végétaux indésirables.

21. L'utilisation des N-arylsulfonyl-N'-triazinyl et -pyrimidinyl-urées de formule I, revendication 1, ou de produits en contenant pour la régulation de la croissance des végétaux.

22. L'utilisation des N-phénylsulfonyl-N'-triazinyl- et -pyrimidinyl-urées de formule I, revendication 1, ou de produits en contenant pour la régulation de la croissance des végétaux cultivés en vue d'une augmentation de rendement.

23. L'utilisation selon la revendication 20, pour la lutte sélective en pré-levée ou en poste-levée contre les mauvaises herbes dans les cultures de végétaux utiles.

24. L'utilisation selon la revendication 23, pour la lutte contre les mauvaises herbes pérennes dans les cultures de végétaux utiles.

25. L'utilisation selon la revendication 21, pour l'inhibition de la croissance des végétaux au-delà du stade 2 feuilles, caractérisée en ce que l'on applique les substances actives en pré-levée.

26. L'utilisation selon la revendication 23, dans les cultures de céréales.